# EUROPEAN PATENT SPECIFICATION

(11) **EP 2 137 539 B1**
(45) Date of publication and mention of the grant of the patent: **06.10.2010**
(21) Application number: 08715633.7
(22) Date of filing: 03.04.2008
(51) Int. Cl.: G01N 33/94, G01N 33/68, C12Q 1/68, A61K 38/00

(54) **AMILORIDE SENSITIVE SODIUM CHANNELS ASSOCIATED WITH PANIC DISORDERS**
MIT PANIKSTÖRUNGEN ASSOZIIERTE AMILORID-SENSITIVE NATRIUMKANÄLE
CANAUX SODIQUES SENSIBLES À L'AMILORIDE ASSOCIÉS À DES TROUBLES DE PANIQUE

(30) Priority: 03.04.2007 DK 200700519
(43) Date of publication of application: 30.12.2009
(73) Proprietor: ILEGUSAVNID, Genetics Resource Centre Minestry of Social Affairs and Health, 100 Torshavn (FO)
(72) Inventor: DAHL, Hans Atli, DK-5330 Munkebo (DK); WANG, August, G., DK-2200 Copenhagen (DK); KRUSE, Torben, DK-5700 Svendborg (DK); VANG, Maria, DK-100 Torshavn (FO); MORS, Ole, DK-8240 Risskov (DK)
(74) Representative: Plougmann & Vingtoft A/S
(86) International application number: PCT/DK2008/050080
(87) International publication number: WO 2008/119360

(56) References cited:
- WO-A-01/81570
- US-A1- 2003 186 860
- WEMMIE JOHN A ET AL: "Overexpression of acid-sensing ion channel 1a in transgenic mice increases acquired fear-related behavior." PROCEEDINGS OF THE NATIONAL ACADEMY OF SCIENCES OF THE UNITED STATES OF AMERICA, vol. 101, no. 10, 9 March 2004 (2004-03-09), pages 3621-3626, XP002445864 ISSN: 0027-8424
- WESTMORELAND PATRICIA ET AL: "The ASIC1a antagonist PcTX-1 reduces fear - related behavior" ACTA PHARMACOLOGICA SINICA, XX, CN, vol. 27, no. Suppl. 1, 1 July 2006 (2006-07-01), page 92, XP008082152 ISSN: 1671-4083
- HETTEMA JOHN M ET AL: "Genetic association study of the amiloride-sensitive cation channel 2 (ACCN2) gene and anxiety spectrum disorders" NEUROPSYCHOPHARMACOLOGY, vol. 31, no. Suppl. 1, December 2006 (2006-12), page S167, XP008082151 & 45TH ANNUAL MEETING OF THE AMERICAN-COLLEGE-OF-NEUROPSYCHOPHARMACOLGY ; HOLLYWOOD, FL, USA; DECEMBER 03 -07, 2006 ISSN: 0893-133X
- HETTEMA JOHN M ET AL: "Lack of association between the amiloride-sensitive cation channel 2 (ACCN2) gene and anxiety spectrum disorders" PSYCHIATRIC GENETICS, vol. 18, no. 2, April 2008 (2008-04), pages 73-79, XP008093635 ISSN: 0955-8829
- BERNARDINELLI LUISA ET AL: "Association between the ACCN1 gene and multiple sclerosis in Central East Sardinia." PLOS ONE 2007, vol. 2, no. 5, 2007, page e480, ISSN: 1932-6203
- BERNARDINELLI LUISA ET AL: "Association between the ACCN1 gene and multiple sclerosis in Central East Sardinia." PLOS ONE 2007, vol. 2, no. 5, 2007, page e480, ISSN: 1932-6203

### SEQUENCE LISTING

<110> Genetics Resource Centre
   Dahl, Hans Atli
   Kruse , Torben
   Vang , Maria
   Mors , Ole
   Wang, August
<120> Amiloride Sensitive Sodium Channels associated with Panic Disorder
<130> 42037PC01
<160> 28
<170> PatentIn version 3.5
<210> 1
   <211> 1135
   <212> DNA
   <213> Homo sapiens
<400> 1
   ggagggagag agactcggat gtggagaaaa ggcagaaata actcagccat ttccacaaga
   60
   ccagctttta atatgactgt gatttgaagc acgaataaat aaatgccaaa gaaaatggaa
   120
   aaaccaaagc aaacctcaga acatcaaaac taaaataaaa aagcataaaa tgaagcaaaa
   180
   caaaacaaat ctccatcgga caaacataaa agccccccta ccccagataa aaagaatcag
   240
   actgtacaag ctgtgtcaca gggagagaag aacgacatgg gcaccatggg tgaacacagg
   300
   cgtcccccac ccgctggctg ctgtgccgcc gtcatcaggc actggggggg agggaggttg
   360
   gcgcgggtca cctttggcgc cgaagcccca ggggcagctt ggcggaaaag ggggtttttg
   420
   ggcaggttaa gaactttcac aaacgacaca agaactgaat gtagcagaga gtcccatggg
   480
   ttccctcttg attgtgcagt tctgattcca gggtggcctc atggtgactt tggacctgga
   540
   atctggtccc actgcacggg cagcagtgtg gacactggaa accgcgtgga ggagtgtcat
   600
   gtacaagaca gacgtggagg tggcgccact aagaagtctg agcatgcagg tgctttatac
   660
   atctggcagt gagatgtgat ggcaggttcg ttcttggaca gttccagagt gtgactcatc
   720
   tctcctaaga gggacgcacg gcggggccca aggatgcgtc gtgttggacg tggccggagc
   780
   gaggtctagg cagctagtct gcaatgtgtg catagggggc tcttgcttct ttccagcact
   840
   ggggccatcc cacctgagct tgctgttcct tgtcctgggt cttgggcctc aaggtctgtt
   900
   tggagggagt gctgggtgac tcgaggggtg tcagcaggca atctcctcca aggtccccag
   960
   ggtcgtctgc aggggcacgt tcacagtgtg actgatggtt tcagagtggt ttggcattgt
   1020
   gtcacaagta ctctggaagg gaagggttgg tgggagttta ttattcgctc agcaaaaatt
   1080
   cttcatgatg ccaccagcgg cccagcctag gccctgggga aggtgctccc cactt
   1135
<210> 2
   <211> 24
   <212> DNA
   <213> Homo sapiens
   <400> 2
   tagtacgctg ctgtgccgcc gtca
   24
<210> 3
   <211> 17
   <212> DNA
   <213> Homo sapiens
<400> 3
   tttggcgccg aagcccc
   17
<210> 4
   <211> 29
   <212> DNA
   <213> Homo sapiens
<400> 4
   ccagtagtac tgggcagctt ggggaaaag
   29
<210> 5
   <211> 29
   <212> DNA
   <213> Homo sapiens
<400> 5
   ccagtagtac tgggcagctt ggggaaaag
   29
<210> 6
   <211> 40
   <212> DNA
   <213> Homo sapiens
<400> 6
   acatgattga ccagtagtac cctgcaaccc tccaccatca
   40
<210> 7
   <211> 18
   <212> DNA
   <213> Homo sapiens
<400> 7
   ggcggggtgg gtgtgtac
   18
<210> 8
   <211> 46
   <212> DNA
   <213> Homo sapiens
<400> 8
   gcagatacat gattgaccag tagtactccc cagcatggta caggtc
   46
<210> 9
   <211> 18
   <212> DNA
   <213> Homo sapiens
<400> 9
   ccctcccccc cagtgcct
   18
<210> 10
   <211> 24
   <212> DNA
   <213> Homo sapiens
<400> 10
   agtacctttt ccccaagctg ccca
   24
<210> 11
   <211> 35
   <212> DNA
   <213> Homo sapiens
   <400> 11
   gaccagtagt acgttcttaa cctgcccaaa aaccc
   35
<210> 12
   <211> 30
   <212> DNA
   <213> Homo sapiens
<400> 12
   tagtacagca cctgcatgct cagacttctt
   30
<210> 13
   <211> 46
   <212> DNA
   <213> Homo sapiens
<400> 13
   tagcagatac atgattgacc agtagtacac agggaccggg cattgg
   46
<210> 14
   <211> 18
   <212> DNA
   <213> Homo sapiens
<400> 14
   aaggcgacca ctcacccc
   18
<210> 15
   <211> 40
   <212> DNA
   <213> Homo sapiens
<400> 15
   catgattgac cagtagtacg ttctccaggc tcaccaccaa
   40
<210> 16
   <211> 19
   <212> DNA
   <213> Homo sapiens
<400> 16
   gctgctgtgc cgccgtcat
   19
<210> 17
<211> 19
<212> DNA
<213> Homo sapiens
<400> 17
   gctgctgtgc cgccgtcac
   19
<210> 18
   <211> 18
   <212> DNA
   <213> Homo sapiens
<400> 18
   tttggcgccg aagcccca
   18
<210> 19
   <211> 18
   <212> DNA
   <213> Homo sapiens
<400> 19
   tttggcgccg aagccccg
   18
<210> 20
   <211> 20
   <212> DNA
   <213> Homo sapiens
<400> 20
   tgggcagctt ggggaaaagg
   20
<210> 21
   <211> 20
   <212> DNA
   <213> Homo sapiens
<400> 21
   tgggcagctt ggggaaaagc
   20
<210> 22
   <211> 20
   <212> DNA
   <213> Homo sapiens
<400> 22
   agacgtggag gtggcgccac
   20
<210> 23
   <211> 20
   <212> DNA
   <213> Homo sapiens
<400> 23
   agacgtggag gtggcgccat
   20
<210> 24
   <211> 17
   <212> DNA
   <213> Homo sapiens
<400> 24
   cggggtgggt gtgtacg
   17
<210> 25
   <211> 17
   <212> DNA
   <213> Homo sapiens
<400> 25
   cggggtgggt gtgtacc
   17
<210> 26
   <211> 17
   <212> DNA
<213> Homo sapiens
<400> 26
   aagggaaaga aggcggc
   17
<210> 27
   <211> 20
   <212> DNA
   <213> Homo sapiens
<400> 27
   acagagcttg aggccaaaag
   20
<210> 28
   <211> 21
   <212> DNA
   <213> Homo sapiens
<400> 28
   ccttgggcaa gtgacttaat c
   21

## Description

### Background

### Panic disorder

Panic disorder (PD) is categorised amongst the anxiety states, which represent the most common psychiatric disorders in society. It is characterised by recurrent attacks of severe anxiety not related to any particular situation or circumstance - they are hence unprovoked and unpredictable (WHO: International Classification of Diseases (ICD), 1993).

The etiology underlying PD is still unknown and presumably complex - involving numerous susceptibility genes as well as environmental factors. Estimates from family- and twin studies ascribe a considerable genetic contribution in the disease etiology of PD. Family studies have shown lifetime prevalence's in first degree relatives between 7.7% and 17.3% (relative risk ranging between 3 and 11). Twin studies have shown probandwise concordance rates among monozygotic twins between 23.9% and 45% while the probandwise concordance rates in dizygotic twins was estimated to be between 10.9% and 15 %. Community studies indicate a lifetime prevalence of PD in the range of 1.2% to 2.4% with a 2-3 fold higher risk among women compared to men.

Among the complex psychiatric disorders, PD comprises a unique feature in that the clinical phenomena can be triggered by pharmacological challenges and the response can be assessed in a clinical setting. Provoking agents like carbon dioxide (Cohen and White, 1972; Gorman et al., 1984), lactate (Pitts, Jr. and McClure, Jr., 1967), Cholecystokinin tetrapeptide (CCK4) (de Montigny, 1989) and Caffeine (Boulenger et al., 1984; Charney et al., 1985) can elicit panic attacks in PD patients. By reproducing the symptoms of panic these agents might reveal parts of the mechanism underlying the patophysiology of anxiety.

The different agents presumably involve different mechanism of action. CCK and Caffeine are both functional neurotransmitters and their role in anxiety might therefore involve the corresponding receptors and related pathways. Further, contrary to carbon dioxide and lactate, CCK and caffeine induced panic attacks activate the Hypothalamic-Pituitary-Adrenal (HPA) axis (Bradwejn et al., 1991; Uhde et al., 1984; Ursin and Olff, 1993).

The mechanism underlying the effect of the two metabolic products CO2 and lactate is, however, more obscure. Klein (Klein, 1993) hypothesised that the triggering of an endogenous "false suffocation alarm" results in activation of the fight or flight mechanism in PD patients. Another theory suggested that the effect of the two metabolic products could be linked to anxiety by a common metabolic "redox challenge" altering the cellular pH level (Carr and Sheehan, 1984). How pH, CO2 and lactate mediate their effect in PD is not clear and no genes in an anxiety related mechanism modulated by these agents have so far been identified.

The present way of diagnosing panic disorder is by the patient's story, which is depending on the patient's recall of events and symptoms. Panic attacks include four of the following: Palpitations, abdominal distress/nausea, numbness/tingling, inability to breathe or shortness of breath, choking, sweating, chest pain, dizziness, depersonalisation, flushes/chills, fear of dying and trembling/shaking.

At present the only other methods used are somatic checks in order to exclude somatic illness such as hearth disease.

Thus, there is a need for other means of diagnosing panic disorder.

As to treatment, there are some short-term benefits with drug therapy, but relapse is common when ceasing drug therapy. Furthermore, some drugs have risk of dependency and some have side effects.

Benzodiazepines may be rapidly effective, but the problems are relapse with discontinuation and risk of drug dependency. Antidepressives (SSRIs and Tricyclics) are generally effective, the problems are relapse with discontinuation and side effects.

In many countries, several of the benzodiazepines as well of the antidepressives are licensed for treatment of panic disorder.

Some efficacy may be gained by unlicensed drugs, such as MAOis, Carbamazepine and Propanolol.

A type of psychotherapy called Cognitive-Behavioural Therapy has some effect and also some extra effect when it is combined with a SSRI. Not all patients can or will participate in psychotherapy and the cost is also a limiting factor.

Thus, there is a need for assays that can provide candidate compounds potentially useful for treatment of panic disorder and obviously also for new therapeutics.

### Amiloride sensitive ion channels

According to the literature the ACCN genes encode a group of acid-sensing ion channels (ASIC's, also referred herein to as amiloride sensitive ion channels), members of the DEG/ENaC superfamily of sodium channels which are ubiquitously distributed in the nervous system (Krishtal, 2003; Krishtal and Pidoplichko, 1981). Four different ASIC genes encoding six polypeptides have been identified so far. The polypeptides form functional channels as tetramers either as homo- or as heteromeric assemblies of the different ASIC subunits. The ACCN1 gene (Price et al., 1996) encodes two polypeptides ASIC2a and ASIC2b both expressed in the brain. The channels are pH sensitive and respond to a decrease in extracellular acidity and the acid induced currents are potentiated by Zn++ and reducing agents (e.g. lactate) (Andrey et al., 2005; Baron et al., 2001). Oxidising agents (Andrey et al., 2005) apart from the ions Ca++ and Mg++ (Immke and McCleskey, 2001) attenuate the sodium currents. The different subunits show different kinetics, permeability, pH sensitivity and respond differently to different modulating agents. ASIC1a seems to be responsible for the effect from reducing agents (Chu et al., 2006) while the Zn++ response is mediated by the ASIC2a subunit (Baron et al., 2001). There are indications that reducing agents might be indirectly involved in the regulation of ASIC activity by chelating Ca++ and Mg++ (Immke and McCleskey, 2001). The metabolically relatively inactive D-enantiomer of lactate also activates the channels (Gorman et al., 1990) indicating that it is unlikely that a lactate metabolite or a metabolic by-product modulates the channel. It is thus more likely that chelation of the divalent ions Ca++ and Mg++ is the mechanism behind lactate activation of ASIC's (Immke and McCleskey, 2001). As carbon dioxide is involved in the cellular pH regulation it might be indirectly involved in regulation of ASIC's in pH dependent manner. Thus, both of the two metabolic challenging agents are probably involved in the regulation of these channels. Compared to the provoking agents, caffeine or CCK, lactate and carbon dioxide do not activate the HPA-axis thereby indicating different mechanism of action, which might very well be acid sensing channels in the CNS. An evolutionary advantage of a swift mechanism of regulation in which the fight or flight response is triggered among our ancestors seems obvious and as pH regulation has one of the steepest regulations in the body (Krishtal, 2003) it might be involved in such an advantageous alarm system. The mechanism of the "false suffocation alarm" suggested by Klein (Klein, 1993) might thus involve the cellular pH level as well as the redox state.

Wemmie et al, 2004 generated mice overexpressing human ASCI1a and showed that overexpressing ASIC1a enhanced fear conditioning, an animal model of acquired anxiety. The authors suggested that ASIC1a and H+-gated currents may contribute to the development of abnormal fear and to anxiety disorders in humans.

With respect to the function of the ASIC's there are likewise connections to the anxiety related pathways. The acid sensing ASIC1a is highly expressed in the Amygdala and is required for Amygdala acid induced currents (Wemmie et al., 2003). This is interesting as the neural plasticity, underlying Pavlovian fear conditioning, is considered to occur in the basolateral complex of the amygdala (Fanselow and LeDoux, 1999). Furthermore ASIC1a-deficient mice showed reduced fear (Wemmie et al., 2003) whereas overexpression of ASIC1a in mice enhanced fear related freezing in conditioning experiments (Wemmie et al., 2004). It is thus likely that ASIC's are involved in the fear conditioning process. Further, the acid induced currents of ASIC's are involved in long term potentiation of the NMDA receptor and considered to be involved in memory and learning (Wemmie et al., 2002). Not only might the mechanism be involved in triggering attacks, it might also be involved in the subsequent development of the anticipatory anxiety. The results indicate a link between conditioned anxiety and ASIC's and by their mode of regulation ASIC's might be the link between the challenging agents, carbon dioxide and lactate, and anxiety.

The above observation relates to general anxiety or fear which is involving several conditions. The studies by Wemmie et al. are all on ASIC1a encoded by the gene ACCN2. The present invention relates to a specific anxiety condition, Panic Disorder.

The individual ASICs proteins or subunits have been known for several years. W0 01/81570 described a novel ASIC complex involving ASIC2a and ASIC3, but it did not link the heteromeric complex to pre-disposition to panic disorder or treatment of panic disorder.

### Summary of the invention

The present inventors have discovered an association of amiloride sensitive ion channels and panic disorder. More specially, the inventors have discovered polymorphisms that associate to a pre-disposition of panic disorder. The discovered polymorphisms are located in the 3'UTR (untranslated region) an ACCN gene, and it is believed that the polymorphisms ultimately affect the cellular activity of the amiloride sensitive ion channel.

Thus, in a first aspect, the invention provides a method of determining a pre-disposition of a subject to panic disorder as defined on the claims. Other aspects of the invention relate to methods of identifying candidate compounds for treatment of panic disorder as defined on the claims.

### Detailed description

### Brief legends of drawings

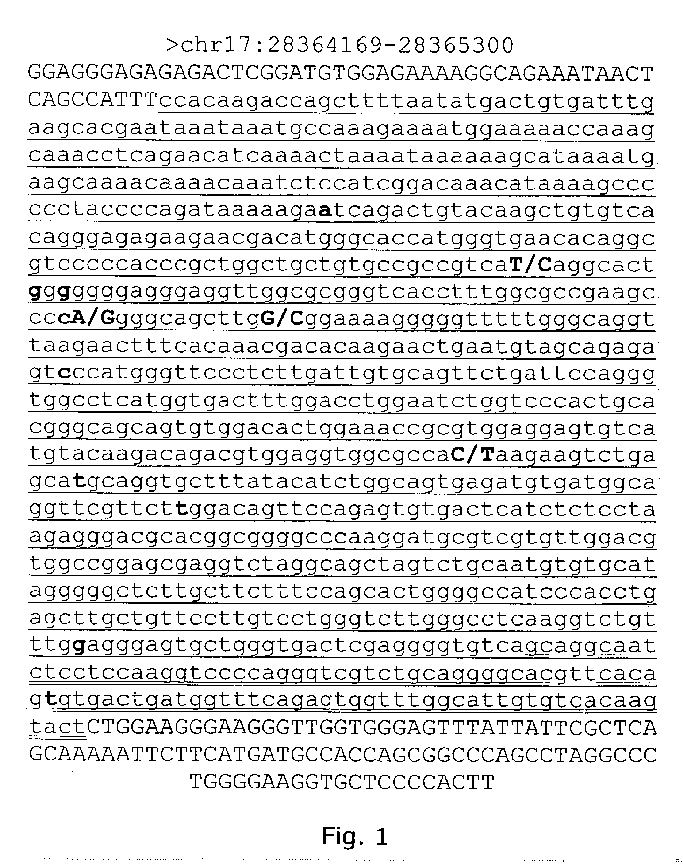

Fig. 1 The 3'UTR of ACCN1. SNPs associated to panic disorders are indicated in bold. Underlined sequence is mRNA. Double underlined sequence is translated mRNA.

### Disclosure of the invention

The present inventors have discovered an association of amiloride sensitive ion channels and panic disorder. More specially, the inventors have discovered polymorphisms that associate to a pre-disposition of panic disorder. The discovered polymorphisms are located in the 3'UTR (untranslated region) of an ACCN gene, and it is believed that the polymorphisms ultimately affect the cellular activity of the amiloride sensitive ion channel.

Thus, a predisposition to panic disorder may be determined by assessing the cellular activity of one or more amiloride sensitive ion channels. The cellular activity is to be understood as the sum of the specific activity of a particular ASIC and the number of the particular ASIC in any given location. The location may e.g. be whole cells, or only the cell membrane. The activity of an ASIC is preferably transport of ions across a membrane.

Consequently, in a first aspect, the present invention provides a method of determining a pre-disposition of a subject comprising
a. determining the apparent cellular activity of a amiloride sensitive ion channel (ASIC) in a sample obtained from the subject
b. determining whether the apparent cellular activity of step a. is different from a corresponding control value, wherein the ASIC is ACCN1.

The term "apparent cellular activity" of an ASIC is used herein for the values of any parameters indicative of the cellular activity of the ASIC. The apparent cellular activity of an ASIC may be determined by analysing the level or physical state of the gene encoding the ASIC, by analysing the level or physical state of the mRNA encoding the ASIC or by analysing the level or physical state of the ASIC protein. Analysing the physical state may include detecting mutations, modifications or location.

As will be clear, the apparent cellular activity may be assessed by measuring e.g. both mRNA level and protein level. In such a case, the apparent cellular activity may be assessed from both measurements, i.e. both measurements contribute to the assessment of the apparent cellular activity.

Preferably, the apparent cellular activity is compared to a control value which is obtained from one or more subjects not being pre-disposed to panic disorder. Obviously, a control value corresponds to the apparent cellular activity such that e.g. an mRNA level (apparent cellular activity) is compared to a control mRNA level and not to a control protein level.

Preferably, a measured cellular activity that is lower than the control value is indicative for the subject being predisposed to panic disorder. In another embodiment, a measured cellular activity that is higher than the control value is indicative for the subject being predisposed to panic disorder.

Subjects not being pre-disposed to panic disorder are individuals that have not ever had any incidents indicative of panic disorder; neither have any close family members of the individuals.

In some embodiments of the invention, the apparent cellular activity is normalised against at least one other cellular marker. Thus, the cellular activity may e.g. be expressed as a ratio of the level of the mRNA encoding the ASIC and another mRNA. Or the cellular activity may be expressed as the ratio of the level of an ASIC protein and the level of another protein.

In a preferred embodiment, the apparent cellular activity of the ASIC is measured as the flux of ions across a barrier, preferably a physiological membrane. Such measurements may e.g. be performed using patch clamp technology.

### Amiloride sensitive ion channels

It is known that ASICs functions as tetramers and heterotetramers exist (e.g comprising combinations of ACCN1, ACCN2, and ACCN3 subunits). Therefore, the present inventors find it highly relevant to not only determine the apparent cellular activity if ACCN1.

Thus, the at least one further ASIC is selected from the group consisting of ACCN2, ACCN3, ACCN4 and ACCN5.

ACCN1 (amiloride-sensitive cation channel 1, neuronal (degenerin)) has genbank accession number: AL834182.

ACCN2 (amiloride-sensitive cation channel 2, neuronal) has genbank accession number: U78181.

ACCN3 (amiloride-sensitive cation channel 3) has genbank accession number: AB010575.

ACCN4 (amiloride-sensitive cation channel 4, pituitary) has genbank accession number:
AJ271643.

ACCN5 (amiloride-sensitive cation channel 5, intestinal) has genbank accession number: AJ252011.

Their sequences (DNA, mRNA and protein) can be retrieved from various databases on the internet, preferably
http://www.ncbi.nlm.nih.gov/entrez/query.fcgi?db=Nucleotide

In the invention, the ASIC is ACCN1, i.e. the apparent cellular activity of ACCN1 is determined.

In a preferred embodiment, the apparent cellular activity of 2, 3, 4 or 5 of the ASICs selected from the group consisting of ACCN2, ACCN3, ACCN4 and ACCN5 is determined in addition to ACCN1.

### mRNA

As indicated above the apparent cellular activity of an ASIC may be determined or assessed at DNA level, RNA level or protein level.

Hence, in one embodiment the cellular activity of an ASIC is assessed by analysing the cellular level of mRNA encoding the ASIC.

The cellular level of mRNA may be determined by qPCR, northern blot or microarray analysis and other methods known to the skilled man.

In another embodiment, the apparent cellular ASIC activity comprises detecting one or more polymorphisms of an ASIC.

As will clear to the skilled artisan, polymorphisms may affect both the expression of the ASIC and the activity of ASIC protein. A polymorphism may e.g. affect transcription of the gene encoding the ASIC, stability of the mRNA coding for the ASIC, translation of the mRNA coding for the ASIC or it could affect the amino acid sequence of the ASIC and thereby affect the activity of the ASIC.

The polymorphisms may be present in the reading frame of the sequence encoding the ASIC, in the 5'UTR of the mRNA encoding the ASIC, in the 3'UTR of the mRNA encoding the ASIC or in the promoter region encoding the ASIC.

The polymorphisms of the ASIC may be detected on DNA level, RNA level or protein level and preferably, the one or more polymorphisms the ASIC is detected on DNA level or RNA level.

As outlined in the examples section, the present inventors have identified polymorphisms in the 3'UTR (untranslated region) of the gene encoding an ASIC. Thus, in another preferred embodiment, the polymorphisms are present in the 3'end UTR of the mRNA encoding the ASIC. Such polymorphisms are not likely to affect the amino acid sequence of the ASIC, but rather the expressed level of the ASIC will be affected.

The polymorphisms may be STRs (short tandem repeats) or more preferably SNPs (single nucleotide polymorphisms). Detection of SNPs can e.g. be done by PCR, optionally qPCR. Also hybridisation based assays may be used.

In a preferred embodiment, the SNP is selected from the group consisting of SNP rs28936, rs28935, rs28933, and finally a previously undescribed SNP at position 28364577 bp according to the UCSC Genomic Browser (March 2006 freeze).

SNPs in the 3'UTR may change the expressed level of the ASIC by affect binding of regulatory RNA binding proteins and/or RNAs such as microRNAs to the 3'UTR or by changing secondary structure leading to changed stability of the mRNA.

### ASIC protein

Instead of analysing the mRNA, the gene product, i.e. ASIC protein may be analysed.

Hence, in one embodiment, the apparent cellular ASIC activity is determined by determining the cellular level of the ASIC protein.

Preferably, determining the cellular level of the ASIC protein comprises a technique selected from the group consisting of immune techniques, mass spectrometry. Thus, immunoblotting or ELISA is suitable means for detecting the cellular level of the ASIC protein.

In another embodiment, the apparent cellular ASIC activity is determined by performing an activity test of an ASIC derived from the subject. The ASIC derived from the subject may be present in a cell extract obtained from the subject. The ASIC derived from the subject may also be prepared by cloning the respective ASIC for subsequent purification and activity tests. Thus, derived in the present context should be interpreted broadly. Preferably, the activity test comprises measuring ion flux across a membrane.

### Kit

Another aspect of the invention is a kit comprising means for determining the cellular activity of one or more amiloride sensitive ion channels as described in the first aspect of the invention. In a preferred embodiment, the kit comprises primers for snp detection, preferably for detection of a snp selected from the group consisting of: SNP rs28936, rs28935, rs28933, and finally a previously undescribed SNP at position 28364577 bp according to the UCSC Genomic

Browser (March 2006 freeze, which corresponds to hg18 and NCBI Build 36.1). See also Bernadinelli L. et al. Plos One 2(s), e480, 2007.

### Pharmaceutical composition

Further disclosed is a pharmaceutical composition comprising an ASIC as described, a peptide derived thereof, a antibody binding to the ASIC, an aptamer binding to the ASIC, a nucleic acid encoding an ASIC, a modulator oligonucleotide (antisense, microRNAs, siRNA), a ASIC specific antibody, amiloride, psalmotoxin I or II, GsMTx4, FMRF-amide or any member of the RF-amide peptide family for treatment of panic disorder.

Instead of using amiloride in the pharmaceutical composition, a chemical compound comprising amiloride may be used. I.e. amiloride may be part of a larger chemical structure that is used.

### Treatment

Yet, another aspect of this disclosure is a method of treatment comprising administering the pharmaceutical composition mentioned above.

A further aspect of the disclosure is use of an ASIC, a peptide derived thereof, an antibody binding to the ASIC, an aptamer binding to the ASIC, a nucleic acid encoding an ASIC, a modulator oligonucleotide (antisense, microRNAs, siRNA), a ASIC specific antibody, amiloride, psalmotoxin I or II, GsMTx4, FMRF-amide or any member of the RF-amide peptide family for treatment of panic disorder.

As will be appreciated, modulating the cellular activity of an ASIC may be used therapeutically, i.e. for treatment or prevention of panic disorder.

Modulating the cellular activity of an ASIC may involve administration of the ASIC per se or a peptide derived thereof. A peptide derived thereof may e.g. be the soluble part of the protein and such soluble part of the protein may act as a decoy.

Also a gene encoding an ASIC may be used for treatment, because introducing the gene into a cell can lead to increased levels of the encoded ASIC in the cell. The cellular activity of an ASIC may also be modulated using a modulator oligonucleotide such as an antisense oligonucleotide, a ribozyme, microRNA or siRNA. Such modulator oligonucleotides can be targeted to the mRNA encoding the ASIC by of way of complementarity between the mRNA and the modulator oligonucleotide.

Further, ligands binding to the ASIC may be used for modulating the cellular activity of the ASIC. Antibodies that bind specifically to the ASIC can be generated by methods known to the skilled man. Also high-affinity aptamers can be generated.

### Identifying candidate compounds

Still another aspect of the invention, is a method of identifying a candidate compound for treatment of panic disorder comprising the steps of
a. Providing a ASIC activity assay
b. Providing a compound
c. Testing the activity of the ASIC in the presence and absence of the compound
d. Verifying whether the compound affects the activity of the ASIC and classify the compound as a candidate compound for the treatment of panic disorder if it is capable of affecting the activity of the ASIC

An identified candidate compound may subsequently be tested in secondary tests for in vivo effects, toxicology and pharmacokinetics and pharmacodynamics.

### Examples

The following example describes how the association of an ASIC (ACCN1) and PD was discovered and supports the association.

### Example 1

### An isolated population

We chose the isolated population of Faroe Islands in the North Atlantic Ocean searching for susceptibility genes in PD. Population isolates have been beneficial in mapping genes and in identifying chromosomal regions for several monogenic disorders. The etiology of complex disorders presumably involves numerous genes and if allelic heterogeneity also applies to the sample the power to detect an association will be considerably reduced. Diminishing the effects of locus- and allelic heterogeneity at the population level is thus of major importance. The experience from the Finnish isolate suggests that heterogeneity was markedly reduced and isolates may hence be advantageous in mapping susceptibility regions in complex disorders. Of major importance are the environmental and genetic homogeneity, a small founding bottleneck followed by stable early phase keeping the population at low number and then a subsequent rapid expansion. In such populations LD might be extended and preserved around a disease locus which increases the chance to detect an association at a relatively large distance from the disease locus, thereby decreasing the number of markers needed to cover a specific region. As a genetic isolate the population of the Faroe Islands seems suitable and meets all the criteria considered beneficial for mapping susceptibility genes in complex disorders. The population history, with respect to genetic studies, has been described previously (Ewald et al. 1999). Population analyses (applying uniparentally transmitted markers) indicate that the population is among the most isolated and presumably is the most genetically homogenous population in the North Atlantic Ocean (Als et al., 2006).

The data presented here is part of a larger study involving a genome wide scan.

### Material and methods

### Clinical Samples

Patients were recruited from the National Hospital in Tórshavn, Faroe Islands. Sixteen treatment seeking individuals agreed to participate and were interviewed by an experienced psychiatrist (AGW) using a brief version of the Present State Examination (Wing et al., 1990). On the basis of the interview a clinical narrative was made for each patient and final diagnosis was made by consensus as a best estimate by a psychiatrist who independently reviewed the clinical narrative and if necessary other relevant material. The diagnoses were made in accordance with the ICD-10, Diagnostic Criteria for Research (WHO: International Classification of Diseases (ICD), 1993) and the diagnostic requirements for inclusion in the study were PD and agoraphobia according to ICD-10. Two of the patients did not fulfil all criteria and a single patient did not deliver a blood sample. The remaining thirteen patients were included in the study. Parental blood samples were also obtained in order to reconstruct the haplotype (in some cases one of the parents was missing, usually the father). Controls were likewise collected as triads. Twenty-two control families unscreened for psychiatric disorders were collected from the same subregion of the Faroe Islands and the parents were used as controls, resulting in a total of 43 control persons.

### Genealogical assessment.

A genealogical search in church and civic records of births, marriages and deaths was made for each patient. Lineages were traced as far back as possible in order to determine relationships between the patients and the shortest possible distance between each pair-wise combination of patients was established. All patients were related to a common ancestor in the early 17th century. On average each pairwise combination of cases found their most recent common ancestor 6.5 generations back, and it was assured that the distance of any two case-individuals to their most recent shared ancestor was at least 3 generations (i.e. 6 meioses separating the two individuals).

### Genotyping and sequencing

DNA was extracted from whole blood using standard sucrose/triton-lysis protocol with sodium chloride/isopropanol precipitation. Primer sequences were obtained from The GDB Human Genome Database (http://gdbwww.gdb.org/) except for a novel microsatellite which was detected in the intron region of ACCN1. This novel marker is located between D17S1540 and D17S2194 at 54.17 cM (http://research.marshfieldclinic.org/genetics/GeneticResearch/compMaps.asp) and is designated D17S1550 in this study. Primer sequences are shown in Table 1. All DNA amplification was performed according to standard PCR procedures either as single marker reactions or as multiplex reactions. PCR products were separated on an ABI 377 Genetic Analyser (Applied Biosystems, Foster City, CA). Alleles where analysed using Genotyper program and stored in the GenBase database for further export to the Genopedigree program (software from Applied Biosystems, Foster City, CA). Pedigree files were checked for Mendelian inheritance.

**Table 1**

| Primer sequences for single base extension (SNaPshot kit, Applied Biosystems) | | |
|---|---|---|
| ID: | Sequence | SNP rs # |
| ex1SNP1F: | **TAGTAC**GCTGCTGTGCCGCCGTCA | rs28936 |
| ex1SNP2F: | TTTGGCGCCGAAGCCCC | rs28935 |
| ex1SNP3F: | **CCAGTAGTAC**TGGGCAGCTTGGGGAAAAG | novel SNP |
| ex1SNP4F: | CCAGTAGTACTGGGCAGCTTGGGGAAAAG | rs28933 |
| ex9SNPF: | **ACATGATTGACCAGTAGTAC**CCTGCAACCCTCCACCATCA | rs9916605 |
| ex10SNP3F: | GGCGGGGTGGGTGTGTAC (18) | rs7214382 |
| ex11SNPF: | **GCAGATACATGATTGACCAGTAGTAC**TCCCCAGCATGGTACAGGTC rs2228990 | |
| ex1SNP1R: | CCCTCCCCCCCAGTGCCT (18) | |
| ex1SNP2R: | **AGTAC**CTTTTCCCCAAGCTGCCCA | |
| ex1SNP3R: | **GACCAGTAGTAC**GTTCTTAACCTGCCCAAAAACCC | |
| ex1SNP4R: | **TAGTAC**AGCACCTGCATGCTCAGACTTCTT | |
| ex9SNPR: | **TAGCAGATACATGATTGACCAGTAGTAC**ACAGGGACCGGGCATTGG | |
| ex10SNP3R: | AAGGCGACCACTCACCCC (18) | |
| ex11SNPR: | **CATGATTGACCAGTAGTA**CGTTCTCCAGGCTCACCACCAA | |

| | | |
|---|---|---|
| "F" in the SNP ID indicates forward primer and "R" indicates reverse primer. | | |

Tail sequence (shown in bold above) was a random typed sequence:
**TAGCAGATACATGATTGACCAGTAGTAC**

Primers sequences for Sybr green allele specific genotyping:

| ID: | Sequence | SNP rs # |
|---|---|---|
| ACCNex1 F1 T: | | |
| | GCTGCTGTGCCGCCGTCAT | rs28936 |
| ACCNex1 F1 C: | | |
| | GCTGCTGTGCCGCCGTCAC | |
| ACCNex1 F2 A: | | |
| | TTTGGCGCCGAAGCCCCA | rs28935 |
| ACCNex1 F2 G: | | |
| | TTTGGCGCCGAAGCCCCG | |
| ACCNex1 F3 G: | | |
| | TGGGCAGCTTGGGGAAAAGG | Novel SNP |
| ACCNex1 F3 C: | | |
| | TGGGCAGCTTGGGGAAAAGC | |
| ACCNex1 F4 C: | | |
| | AGACGTGGAGGTGGCGCCAC | rs28933 |
| ACCNex1 F4 T: | | |
| | AGACGTGGAGGTGGCGCCAT | |
| ACCNex10 F G: | | |
| | CGGGGTGGGTGTGTACG | rs7214382 |
| ACCNex10 F C | | |
| | CGGGGTGGGTGTGTACC | |
| ACCNex10 R G/C | | |
| | AAGGGAAAGAAGGCGGC | |

### Novel microsatellite:

| ID: | Sequence | Dye color | Size range |
|---|---|---|---|
| D17S1550 I: | ACAGAGCTTGAGGCCAAAAG | 6-FAM | 206-230 |
| bp. | | | |
| D17S1550 II: | | CCTTGGGCAAGTGACTTAATC | |

(primer I location: bp. 28639752 - 28639771 and primer II location: bp. 28639956 - 28639976 according to UCSC genomic browser March 2006 freeze, which corresponds to hg18 and NCBI Build 36.1)

### ACCN1 sequencing.

Exons and exon-intron boundaries of the ACCN1 gene where amplified under standard PCR procedures and subsequently sequenced with the ABI Big Dye Terminator 3.1 kit as recommended by the manufacturer (Applied Biosystems, Foster City, CA). Fourteen individuals (13 case individuals and 1 control individual) were sequenced in order to detect SNP's in the gene. Nine SNP's where detected in the gene of which 7 were informative. Six of them are known SNP's and listed in the SNP database, these were rs28936, rs28935, rs28933, rs9916605, rs7214382 and rs2228990 and finally a previously undescribed SNP at position 28364577 bp according to the UCSC Genomic Browser (march 2006 freeze) See also Bernadinelli L. et al. Plos one 2(S): e480, 2007. The novel SNP is designated SNP3 in this study.

### SNP genotyping.

Genotyping of ACCN1 SNP's was performed with the SNaPshot kit from Applied Biosystems modified by scaling volumes down to half compared to manufactures prescriptions. The primer sequences for SNaPshot typing were 17-25-mers on each side of the SNP to allow forward and reverse single base extension (SBE) genotyping. A random sequence tale was added to some of them to allow multiplex loading for genotyping on the ABI 377 Genetic Analyser (Applied Biosystems, Foster City, CA) (primer sequences and tale sequences are listed in table 1). Varying degrees of discrepancy in genotyping outcome were observed at SNP's rs28936, rs28935, rs28933, rs7214382, and the novel SNP3 depending on the direction of typing and likewise also discrepancies with some of the result from the initial sequencing. A secondary genotyping setup was thus applied to these SNP's using allele specific primers and amplified with the use of the Cyber Green kit in accordance with the prescriptions provided by the manufacturer (Applied Biosystems, Foster City, CA; Primer sequences are listed in table 1). The reactions were amplified and detected on the Real Time 7700 (Applied Biosystems, Foster City, CA), each reaction was performed in doublet with the following settings: 10 min. at 95° C followed by 40 cycles at 95° C for 15 sek. and 67,3° C. for 1 min. Allele specific amplification seemed to be the best method to detect the variations.

### Statistical considerations.

Genotypes from the chromosome wide scan were tested for association using Monte Carlo based tests as implemented in CLUMP (Sham and Curtis, 1995). CLUMP is especially useful when analysing highly polymorphic markers in small sample size studies where few observations are expected for some alleles or haplotypes. The CLUMP statistics presented in this paper are from the subtests T1 and T4. The T1 value is the standard Pearson Chi-square test on the raw 2 x N contingency table and T4 value is obtained by reshuffling the columns of the raw 2 x N table into new 2 x 2 tables until the Chi-square value has reached a maximum. The analyses were performed on single marker allele counts and on two-locus haplotype counts. In order to compensate for multiple testing and reduce the type I error rate, the significance threshold was set to 0.005 for single-locus analysis and 0.01 for two-locus analysis. The most interesting alleles or haplotypes producing P-values below the threshold were tested for significance in Fisher's exact test to see if specific allele or haplotype preferentially displayed a skewed distribution. Markers showing significant data were flanked by additional markers in order to further delineate the IBD status of the region. As individuals of the case group are distantly related there is increased likelihood that individual share haplotypes inherited identical by descent by chance (IBD0) from a common ancestor, haplotypes not necessarily harbouring a disease allele. Thus the probability that the affected individuals share a certain IBD0 haplotype from a known ancestor through a specific genealogical relationship was calculated by use of the formula given by (Houwen et al., 1994).

### Population structure analyses.

In case-control association studies it is important that the samples under study come from the same genetic background and share the same population history. Ignored population structures or improperly accounted relatedness among individuals can lead to increased type I error rate and inflated p-values (Bourgain and Genin, 2005). The controls of this study are randomly selected while cases are selected on the background of disease status and we might, unintentionally, have increased both the degree of pairwise relatedness and inbreeding in the case group, the latter because inbreeding can increase disease prevalence (Rudan et al., 2003). On the other hand, if cases and controls are matched inbreeding can increase the power to detect susceptibility loci (Genin and Clerget-Darpoux, 1996). To asses the level of relatedness and inbreeding we applied methods to investigate both population structure and inter- and intra-individual relationships. Analyses were based on genome-wide multilocus data from 78 more or less unlinked markers from the present ongoing genome scan - the average marker-spacing was 53 cM.

Genetic differentiation and population structure were evaluated by Wright's F-statistics. Under the hypothesis of no differentiation between the individuals and the subpopulations (i.e. case and control groups), a null distribution of F_{IT}, F_{IS} and F_{ST} values was obtained by performing 3000 permutations among all individuals (F_{IT}), among individuals within the case-control populations (F_{IS}) and among the case-control populations (F_{ST}) as implemented in SPAGEDi 1.2 (Hardy and Vekemans, 2002). Further, Bayesian model-based clustering as implemented in STRUCTURE (Pritchard et al., 2000), was applied to in order to infer any potential cryptic substructure in the data set. Models with and without admixture were applied without using any prior information on population structure (i.e. disease status). A burnin length of 100,000 simulations, followed by 1,000,000 simulations was used to get accurate parameter estimates.

The degree of inter- and intra individual correlations was evaluated by estimating kinship coefficients (k*ᵢⱼ*) (Loiselle et al., 1995) for each pairwise combination of individuals in addition to intra-individual inbreeding coefficients as implemented in SPAGEDi 1.2 (Hardy and Vekemans, 2002). Within- and between-groups averages of pairwise kinship coefficients were compared. To further reveal any genetic subclustering pair-wise genetic distance was calculated according to Rousset (Rousset, 2000) as implemented in SPAGEDi 1.2 (Hardy and Vekemans, 2002) and the estimates were used in a multidimensional scaling (SPSS 11.5) to map similarity between individuals relative to each other.

### Results

### Analyses of genetic relatedness and population structure

In summary Wright's F statistics did not reveal any signs of genetic differentiation between cases and controls (F_{ST}) and likewise no difference on the individual level neither when comparing relative to the total sample (F_{IT}) nor relative to the sub samples (F_{IS}) (data not shown). The cluster analysis implemented in STRUCTURE (Pritchard et al., 2000) found it more likely that the data belonged to a single cluster than to multiple clusters (data not shown). Likewise the multidimensional scaling of Rousset's genetic distances did not show any clustering of cases compared to controls (data not shown).

Average within and between group kinship coefficients did not differ significantly indicating that cases are not closer related to each other than the are to controls and vice versa. Some specific pairs of individuals revealed relatively high pair-wise kinship coefficient but these occurred equally frequent within the case group, within the control group as well as between the groups. Such cryptic relatedness might increase the type I error rate but as these seem randomly distributed they should do so in both directions. Since we have detailed knowledge of the genealogy of cases we estimated the likelihood for chance observations of specific haplotype frequencies within the case group (Table 3).

In conclusion we did not observe any significant stratification and the samples might thus be considered sub-samples with the same genetic background.

### The chromosome 17q11.2 region

Data from an initial 10 cM chromosome wide scan of chromosome 17 showed P-values below a predefined threshold of 0.01 in the two locus CLUMP analysis involving segment D17S1294-D17S1293 (data not shown). The two-marker segment was located in the 50-56 cM region on chromosome 17q spanning amongst other, the SLC6A4 gene encoding the serotonin transporter. In order to further delineate the IBD status of this region the marker density was increased. The data are presented in Table 2.

**Table 2 Empirical p-values obtained from CLUMP (Sham and Curtis 1995) on the distribution of single marker alleles and two-locus haplotype frequencies on chromosome 17.**

| | gle locus | | Two-loci | | | |
|---|---|---|---|---|---|---|
| | Positon cM | | | | | Intermarke |
| Marker | Marshmed | T1 | T4 | T1 | T4 | distance cM |
| | D17S1308 | | .6 | 0.209 | 0.183 | |
| D17S1298 | 10.7 | 0.171 | 0.166 | 0.032 | 0.093 | 10.1 |
| D17S796 | 14.7 | 0.355 | 0.185 | 0.114 | 0.075 | 4.0 |
| D17S974 | 22.2 | 0.022 | 0.052 | 0.201 | 0.188 | 7.6 |
| D17S1303 | 23.6 | 0.067 | 0.039 | 0.085 | 0.038 | 1.3 |
| D17S921 | 36.1 | 0.447 | 0.164 | 0.391 | 0.341 | 12.6 |
| D17S122 | 41.1 | 0.730 | 0.812 | 0.917 | 0.868 | 5.0 |
| D17S783 | 47.0 | 0.581 | 0.583 | 0.863 | 0.899 | 5.9 |
| D17S1873 | 50.2 | 0.126 | 0.038 | 0.117 | 0.047 | 3.2 |
| D17S1294 | 50.7 | 0.412 | 0.429 | 0.321 | 0.265 | 0.5 |
| SERT s/l | 50.7 | 0.478 | 0.478 | 0.389 | 0.499 | 0.0 |
| D17S1863 | 50.7 | 0.497 | 0.428 | 0.374 | 0.376 | 0.0 |
| D17S1800 | 51.6 | 0.770 | 0.700 | 0.833 | 0.837 | 0.9 |
| D17S1880 | 53.4 | 0.461 | 0.303 | 0.187 | 0.231 | 1.8 |
| D17S798 | 53.4 | 0.418 | 0.586 | 0.371 | 0.298 | 0.0 |
| rs28936 | 53.4 | **0.003** | **0.003** | 0.047 | 0.061 | 0.0 |
| rs28935 | 53.4 | **0.001** | **0.001** | **0.0025** | **0.0028** | 0.0 |
| Novel SNP | 53.4 | 0.014 | 0.014 | **0.0033** | **0.0033** | 0.0 |
| rs28933 | 53.4 | **0.001** | **0.001** | **0.0006** | **0.0002** | 0.0 |
| D17S1540 | 54.2 | 0.018 | 0.020 | **0.0007** | **0.0008** | 0.8 |
| rs9916605 | 54.2 | 1.000 | 1.000 | 0.058 | 0.044 | 0.0 |
| D17S1550 | 54.2 | 0.467 | 0.390 | 0.465 | 0.352 | 0.0 |
| rs7214382 | 54.2 | 0.476 | 0.476 | 0.438 | 0.419 | 0.0 |
| D17S2194 | 54.2 | 0.118 | 0.121 | 0.181 | 0.385 | 0.0 |
| D17S1850 | 55.3 | 0.109 | 0.171 | 0.296 | 0.225 | 1.2 |
| rs2228990 | 56.0 | 0.521 | 0.521 | 0.118 | 0.185 | 0.7 |
| D17S1293 | 56.5 | 0.148 | 0.130 | 0.115 | 0.214 | 0.5 |
| D17S1842 | 57.7 | 0.067 | 0.059 | **0.007** | **0.007** | 1.2 |
| D17S1872 | 57.7 | 0.341 | 0.388 | 0.340 | 0.419 | 0.0 |
| D17S933 | 57.7 | 0.715 | 0.627 | 0.259 | 0.228 | 0.0 |
| D17S1299 | 62.0 | 0.344 | 0.380 | 0.236 | 0.261 | 4.3 |
| D17S943 | 68.4 | 0.837 | 0.901 | 0.052 | 0.194 | 6.4 |
| D17S809 | 74.5 | 0.565 | 0.424 | 0.277 | 0.335 | 6.0 |
| D17S1606 | 80.4 | 0.259 | 0.238 | **0.00050** | **0.00030** | 5.9 |
| D17S1290 | 82.0 | 0.732 | 0.813 | 0.148 | 0.146 | 1.6 |
| D17S1297 | 83.4 | 0.445 | 0.550 | 0.889 | 0.957 | 1.4 |
| D17S1816 | 85.9 | 0.173 | 0.141 | 0.269 | 0.445 | 2.5 |
| D17S1813 | 88.1 | 0.638 | 0.321 | 0.215 | 0.169 | 2.1 |
| D17S940 | 89.9 | 0.198 | 0.422 | 0.606 | 0.596 | 1.8 |
| D17S2059 | 93.3 | 0.081 | 0.110 | 0.044 | 0.258 | 3.4 |
| D17S1862 | 97.6 | 0.018 | 0.051 | **0.002** | 0.003 | 4.3 |
| D17S801 | 103.5 | 0.780 | 0.763 | 0.075 | 0.040 | 5.9 |
| D17S1847 | 111.2 | 0.903 | 0.903 | 0.846 | 0.976 | 7.7 |
| D17S1830 | 116.9 | 0.303 | 0.148 | 0.018 | 0.050 | 5.6 |
| D17S784 | 116.9 | 0.485 | 0.440 | 0.372 | 0.250 | 0.0 |
| D17STFZ1 | 122.0 | 0.811 | 0.735 | 0.742 | 0.745 | 5.1 |
| D17STFZ2 | 124.0 | 0.547 | 0.481 | 0.882 | 0.925 | 2.0 |
| D17S668 | 126.5 | 0.473 | 0.384 | 0.600 | 0.561 | 2.5 |
| D17S928 | 126.5 | 0.209 | 0.242 | 0.688 | 0.903 | 0.0 |

Markers from the initial genome wide scan are shown in bold. P-values below a specific significance threshold are also shown in bold. Thresholds were as follows p≤0.005 for single marker analyses and p≤0.01 for two-locus analyses. T1 calculations are based on the raw 2 x N table while T4 is obtained by clumping columns of the raw 2 x N table into a new 2 x 2 table where allele counts are reshuffled until χ-square value is maximised (Sham and Curtis 1995).

**Table 3 Alleles and segments of most interest**

| | | Observed Frequency | | Fisher's exact | IBD by chance | |
|---|---|---|---|---|---|---|
| Segment | Haplotype | Cases | Controls | (P) | (P) IBD₀ | (P) IBD₀₋ₛᵤₘ |
| **2- and 3-locus in the 53-56 cM region** | | | | | | |
| rs28935-SNP3 | *2;1* | 9/26 | 12/86 | 0.041 | 2.587 x10⁻¹¹ | 5.493 x 10⁻⁹ |
| SNP3- rs28933 | *1;1* | 15/26 | 18/84 | **0.0011** | 1.230 x10⁻²² | 2.673 x10⁻¹⁹ |
| rs28933-D17S1540 | *1;190* | 8/18 | 4/72 | **0.00018** | 3.521 x10⁻¹¹ | 5.615 x10⁻⁹ |
| D17S2194-1850 | *204; 272* | 12/24 | 21/72 | 0.083 | 1.285 x10⁻¹⁷ | 2.028 x10⁻¹⁴ |
| D17S1293-1842 | *262;187* | 4/18 | 1/86 | **0.003** | 1.179 x10⁻⁴ | 0.0103 |
| D17S1842-1872 | *187;130* | 4/22 | 16/84 | 1.000 | 3.718 x10⁻⁴ | 0.0235 |
| | *187;114* | 3/22 | 5/84 | 0.358 | 0.0071 | 0.3954 |
| rs28936- rs28935-SNP3 | *2;2;1* | 8/20 | 9/76 | **0.0069** | 9.940x10⁻¹¹ | 1.541 x10⁻⁸ |
| rs28935-SNP3- rs28933 | **2;1;1** | 8/18 | 5/78 | **0.00025** | 3.521 x10⁻¹¹ | 5.501 x10⁻⁹ |
| | *1;2;2* | 2/18 | 2/78 | 0.158 | 0.0664 | 1.000 |
| SNP3-rs28933-D17S1540 | *1;1;190* | 8/20 | 4/70 | **0.00049** | 9.940 x10⁻¹¹ | 1.541 x10⁻⁸ |
| **2-locus in the 95 cM region.** | | | | | | |
| D17S809-D17S1606 | *241;5* | 4/20 | 0/78 | **0.0025** | 1.147 x10⁻⁴ | 0.0335 |
| | *239;5* | 4/20 | 0/78 | **0.0025** | 1.147 x10⁻⁴ | 0.0335 |
| | *239;1* | 2/20 | 0/78 | 0.0536 | 0.0546 | 1.000 |

Specific haplotypes are tested for association in the Fisher's exact test. IBD calculations are performed according to the formula given by Houwen et al. (Houwen et al. 1994). (P) IBD₀ indicates the probability that the given haplotype is inherited by chance with its observed frequency among 13 cases related to a shared ancestor living 6.5 generations ago. The IBD₀₋ₛᵤₘ column shows the same probability summed over all 29 marker in the chromosome-wide analysis, due to the conservative way of calculation IBD₀ this value can sometimes exceed 1.000.

Low P-values were observed on the q arm in the 56-57 cM region at the two locus segment D17S1293-D17S1842 (Table 2). Fisher's exact test confirmed the overrepresentation of one particular haplotype (262-187; Table 3). The 262-187 haplotype might also be present in the paternally transmitted case chromosomes of individuals D and E but as the phase of the 187 allele was unknown these were not included in the statistical evaluation. The aggregation of presumable extended haplotypes containing the 262-187 haplotype of markers D17S1293-D17S1842. The likelihood that this segment was inherited IBD0 by chance among 4 out of 18 case chromosomes with the given genealogy was relatively high when summed over all markers (Table 3). Because of the observed haplotype pattern in this region and because of the possibility that not 4 but 6 chromosomes carried the specific haplotype we decided to investigate it further in spite of the relatively high IBD0 likelihood.

The D17S1293-1842 segment is located distal to the ACCN1 gene encoding an amiloride sensitive sodium channel highly expressed in brain regions known to be involved in anxiety-modulation (Fanselow and LeDoux, 1999; Wemmie et al., 2003). Hence, the whole ACCN1 transcript including exon-intron boundaries was sequenced in order to identify susceptibility variants and additional microsatellites within this region were tested. Seven of the identified SNP's were considered informative for further analyses and these were genotyped for the whole sample. The statistical analyses of the SNP genotypes revealed 3 SNP's in the 3' UTR of the ACCN1 gene displaying significant association to PD in the single locus analysis (Table 2). Four closely spaced SNP's in the 3' UTR and the adjacent distal marker D17S1540 displayed significant results in the two-locus analyses (Table 2). The two-loci were fractions of the same haplotype (2-2-1-1-190) of the 5 marker segment between rs28936 and D17S1540 apparently being very common among case individuals as revealed by Fisher's exact tests (Table 3). The chance that the observed segments in the case group are inherited IBD by chance through the known genealogy appeared very unlikely according to the formula given by Houwen et al (Houwen et al., 1994) (Table 3). Based on the above results we suggest that ACCN1 gene is involved in the pathophysiology of PD.

### Conclusion

In the present study significant association was observed between SNP's in the 3' UTR of the ACCN1 gene and PD and also between adjacent microsatellite markers distal to the 5'end of the ACCN1 gene and PD. It is highly likely that the observed association is disease related and that ACCN1 is involved in increasing the susceptibility to PD.

### Reference List

Als,T.D., Jorgensen,T.H., Borglum,A.D., Petersen,P.A., Mors,O., and Wang,A.G. (2006). Highly discrepant proportions of female and male Scandinavian and British Isles ancestry within the isolated population of the Faroe Islands. Eur. J. Hum. Genet. 14, 497-504.
Andrey,F., Tsintsadze,T., Volkova,T., Lozovaya,N., and Krishtal,O. (2005). Acid sensing ionic channels: modulation by redox reagents. Biochim. Biophys. Acta 1745, 1-6.
Baron,A., Schaefer,L., Lingueglia,E., Champigny,G., and Lazdunski,M. (2001). Zn2+ and H+ are coactivators of acid-sensing ion channels. J. Biol. Chem. 276, 35361-35367.
Boulenger,J.P., Uhde,T.W., Wolff,E.A., III, and Post,R.M. (1984). Increased sensitivity to caffeine in patients with panic disorders. Preliminary evidence. Arch. Gen. Psychiatry 41, 1067-1071.
Bourgain,C. and Genin,E. (2005). Complex trait mapping in isolated populations: Are specific statistical methods required? Eur. J. Hum. Genet. 13, 698-706.
Bradwejn,J., Koszycki,D., and Shriqui,C. (1991). Enhanced sensitivity to cholecystokinin tetrapeptide in panic disorder. Clinical and behavioral findings. Arch. Gen. Psychiatry 48, 603-610.
Carr,D.B. and Sheehan,D.V. (1984). Panic anxiety: a new biological model. J. Clin. Psychiatry 45, 323-330.
Charney,D.S., Heninger,G.R., and Jatlow,P.I. (1985). Increased anxiogenic effects of caffeine in panic disorders. Arch. Gen. Psychiatry 42, 233-243.
Chu,X.P., Close,N., Saugstad,J.A., and Xiong,Z.G. (2006). ASIC1a-specific modulation of acid-sensing ion channels in mouse cortical neurons by redox reagents. J. Neurosci. 26, 5329-5339.
Cohen,M.E. and White,P.D. (1972). Neurocirculatory asthenia: 1972 concept. Mil. Med. 137, 142-144.
de Montigny,C. (1989). Cholecystokinin tetrapeptide induces panic-like attacks in healthy volunteers. Preliminary findings. Arch. Gen. Psychiatry 46, 511-517.
Ewald,H., Wang,A.G., Vang,M., Mors,O., Nyegaard,M., and Kruse,T.A. (1999). A haplotype-based study of lithium responding patients with bipolar affective disorder on the Faroe Islands. Psychiatr. Genet. 9, 23-34.
Fanselow,M.S. and LeDoux,J.E. (1999). Why we think plasticity underlying Pavlovian fear conditioning occurs in the basolateral amygdala. Neuron 23, 229-232.
Genin,E. and Clerget-Darpoux,F. (1996). Association studies in consanguineous populations. Am. J. Hum. Genet. 58, 861-866.
Gorman,J.M., Askanazi,J., Liebowitz,M.R., Fyer,A.J., Stein,J., Kinney,J.M., and Klein,D.F. (1984). Response to hyperventilation in a group of patients with panic disorder. Am. J. Psychiatry 141, 857-861.
Gorman,J.M., Goetz,R.R., Dillon,D., Liebowitz,M.R., Fyer,A.J., Davies,S., and Klein,D.F. (1990). Sodium D-lactate infusion of panic disorder patients. Neuropsychopharmacology 3, 181-189.
Hardy,O.J. and Vekemans,X. (2002). SPAGEDi: a versatile computer program to analyse spatial genetic structure at the individual or population levels. Mol. Ecol. Notes 2, 618-620.
Houwen,R.H., Baharloo,S., Blankenship,K., Raeymaekers,P., Juyn,J., Sandkuijl,L.A., and Freimer,N.B. (1994). Genome screening by searching for shared segments: mapping a gene for benign recurrent intrahepatic cholestasis. Nat. Genet. 8, 380-386.
Immke,D.C. and McCleskey,E.W. (2001). Lactate enhances the acid-sensing Na+ channel on ischemia-sensing neurons. Nat. Neurosci. 4, 869-870.
Klein,D.F. (1993). False suffocation alarms, spontaneous panics, and related conditions. An integrative hypothesis. Arch. Gen. Psychiatry 50, 306-317. Krishtal,O. (2003). The ASICs: signaling molecules? Modulators? Trends Neurosci. 26, 477-483.
Krishtal,O.A. and Pidoplichko,V.I. (1981). A receptor for protons in the membrane of sensory neurons may participate in nociception. Neuroscience 6, 2599-2601.
Loiselle,B.A., Stork,V.L., Nason,J., and Graham,C. (1995). Spatial Genetic-Structure of A Tropical Understory Shrub, Psychotria Officinalis (Rubiaceae). American Journal of Botany 82, 1420-1425.
Pitts,F.N., Jr. and McClure,J.N., Jr. (1967). Lactate metabolism in anxiety neurosis. N. Engl. J. Med. 277, 1329-1336.
Price,M.P., Snyder,P.M., and Welsh,M.J. (1996). Cloning and expression of a novel human brain Na+ channel. J. Biol. Chem. 271, 7879-7882.
Pritchard,J.K., Stephens,M., and Donnelly,P. (2000). Inference of population structure using multilocus genotype data. Genetics 155, 945-959.
Rousset,F. (2000). Genetic differentiation between individuals. J. Evol. Biology 13, 58-62.
Sham,P.C. and Curtis,D. (1995). Monte Carlo tests for associations between disease and alleles at highly polymorphic loci. Ann. Hum. Genet. 59 ( Pt 1), 97-105.
Uhde,T.W., Boulenger,J.P., Jimerson,D.C., and Post,R.M. (1984). Caffeine: relationship to human anxiety, plasma MHPG and cortisol. Psychopharmacol. Bull. 20, 426-430.
Ursin,H. and Olff,M. (1993). The stress response. In Stress: From Synapse to Syndrome, S.Stanford and P.Salmon, eds. (New York: Academic Press).
Wemmie,J.A., Askwith,C.C., Lamani,E., Cassell,M.D., Freeman,J.H., Jr., and Welsh,M.J. (2003). Acid-sensing ion channel 1 is localized in brain regions with high synaptic density and contributes to fear conditioning. J. Neurosci. 23, 5496-5502.
Wemmie,J.A., Chen,J., Askwith,C.C., Hruska-Hageman,A.M., Price,M.P., Nolan,B.C., Yoder,P.G., Lamani,E., Hoshi,T., Freeman,J.H., Jr., and Welsh,M.J. (2002). The acid-activated ion channel ASIC contributes to synaptic plasticity, learning, and memory. Neuron 34, 463-477.
Wemmie,J.A., Coryell,M.W., Askwith,C.C., Lamani,E., Leonard,A.S., Sigmund,C.D., and Welsh,M.J. (2004). Overexpression of acid-sensing ion channel 1a in transgenic mice increases acquired fear-related behavior. Proc. Natl. Acad. Sci. U. S. A 101, 3621-3626.
WHO: International Classification of Diseases (ICD).
   http://www.who.int/classifications/icd/en/ . 1993.
Wing,J.K., Babor,T., Brugha,T., Burke,J., Cooper,J.E., Giel,R., Jablenski,A., Regier,D., and Sartorius,N. (1990). SCAN. Schedules for Clinical Assessment in Neuropsychiatry. Arch. Gen. Psychiatry 47, 589-593.

## Claims

1. A method of determining a pre-disposition to panic disorder of a subject comprising
a. determining the apparent cellular activity of ACCN1 in a sample obtained from the subject,
b. determining whether the apparent cellular activity of step a. is different from a corresponding control value, -wherein the apparent cellular activity of ACCN1 is determined by one or more of the following
i. analysing the cellular level of mRNA encoding ACCN1
ii. analysing the cellular level of the ACCN1 protein
iii. detecting one or more polymorphisms of an ACCN1.

2. The method of claim 1, wherein the control value is obtained from one or more suspects not being pre-disposed to panic disorder.

3. The method of any of the preceding claims, wherein the apparent cellular activity is normalised against another cellular marker.

4. The method of any of the preceding claims, wherein the cellular level of mRNA is determined by qPCR, northern blot or microarray analysis.

5. The method of any of claims 1-3, wherein one or more polymorphisms of ACCN1 are detected on DNA level, RNA level or protein level.

6. The method of claim 5, wherein one or more polymorphisms of ACCN1 is detected on DNA level or RNA level.

7. The method of claim 6, wherein the polymorphisms is present in the 3'end UTR of the ASIC.

8. The method of any of claims 6 and 7, wherein the polymorphism is a SNP.

9. The method of claim 8, wherein the SNP is selected from the group consisting of: SNP rs28936, rs28935, rs28933, and a SNP at position 28364577 bp according to the UCSC Genomic Browser March 2006 freeze.

10. The method of any of claims 1-3, wherein analysing the cellular level of the ACCN1 protein comprises a technique selected from the group consisting of immune techniques and mass spectrometry.

11. A method of identifying candidate compound for treatment of panic disorder comprising the steps of
a. Providing a ACCN1 activity assay
b. Providing a compound
c. Testing the activity of the ACCN1 in the presence and absence of the compound
d. Verifying whether the compound affects the activity of the ACCN1 and classify the compound as a candidate compound if it is capable of affecting the activity of the ACCN1.

## Patentansprüche

1. Verfahren zur Bestimmung einer Prädisposition für Panikstörung bei einer Person, umfassend
a. Ermitteln der offensichtlichen Zellaktivität von ACCN1 in einer von der Person genommenen Probe,
b. Ermitteln, ob sich die offensichtliche Zellaktivität aus Schritt a. von einem entsprechenden Kontrollwert unterscheidet,
- wobei die offensichtliche Zellaktivität von ACCN1 durch eines oder mehrere der Folgenden ermittelt wird
i. Analyse des Gehalts an für ACCN1 kodierender mRNA auf Zellebene
ii. Analyse des Gehalts an ACCN1-Protein auf Zellebene
iii. Erfassen eines oder mehrerer Polymorphismen eines ACCN1

2. Verfahren nach Anspruch 1, wobei der Kontrollwert von einer oder mehreren Personen erhalten wird, die keine Prädisposition für Panikstörung haben.

3. Verfahren nach einem der vorherigen Ansprüche, wobei die offensichtliche Zellaktivität im Verhältnis zu einem anderen Zellmarker normalisiert wird.

4. Verfahren nach einem der vorherigen Ansprüche, wobei der Gehalt an mRNA auf Zellebene mittels qPCR, Northern Blot oder Mikroarray-Analyse ermittelt wird.

5. Verfahren nach einem der Ansprüche 1-3, wobei einer oder mehrere Polymorphismen von ACCN1 auf DNA-Ebene, RNA-Ebene oder Proteinebene erfasst werden.

6. Verfahren nach Anspruch 5, wobei einer oder mehrere Polymorphismen von ACCN1 auf DNA-Ebene oder RNA-Ebene erfasst werden.

7. Verfahren nach Anspruch 6, wobei die Polymorphismen am UTR des 3'-Endes von ASIC vorhanden sind.

8. Verfahren nach einem der Ansprüche 6 und 7, wobei der Polymorphismus ein SNP ist.

9. Verfahren nach Anspruch 8, wo das SNP ausgewählt ist aus der Gruppe, bestehend aus: SNP rs28936, rs28935, rs28933 und einem SNP an Position 28364577 bp gemäß UCSC Genomic Browser March 2006 freeze.

10. Verfahren nach einem der Ansprüche 1-3, wobei die Analyse des Gehalts an ACCN1-Protein auf Zellebene eine Technik umfasst, ausgewählt aus der Gruppe, bestehend aus Immuntechniken und Massenspektrometrie.

11. Verfahren zum Identifizieren einer möglichen Verbindung zur Behandlung der Panikstörung, umfassend die Schritte
a. Bereitstellen eines ACCN1-Aktivitätsassays
b. Bereitstellen einer Verbindung
c. Testen der Aktivität von ACCN1 in Gegenwart und Abwesenheit der Verbindung
d. Bestätigen, ob die Verbindung die Aktivität von ACCN1 beeinflusst und Klassifizieren der Verbindung als mögliche Verbindung, wenn sie die Aktivität von ACCN1 zu beeinflussen vermag.

## Revendications

1. Méthode de détermination d'une prédisposition à un trouble panique chez un sujet comprenant les étapes consistant à
a. déterminer l'activité cellulaire apparente d'ACCN1 dans un échantillon provenant du sujet,
b. déterminer si l'activité cellulaire apparente de l'étape a est différente d'une valeur témoin correspondante,
où l'activité cellulaire apparente d'ACCN1 est déterminée par au moins l'une des méthodes suivantes
i. analyse du niveau cellulaire d'ARN messager codant pour ACCN1
ii. analyse du niveau cellulaire de protéine ACCN1
iii. détection d'au moins un polymorphisme d'un ACCN 1.

2. La méthode de la revendication 1, dans laquelle la valeur témoin provient d'au moins un suspect non prédisposé à un trouble panique.

3. La méthode de l'une quelconque des revendications précédentes, dans laquelle l'activité cellulaire apparente est normalisée par rapport à un autre marqueur cellulaire.

4. La méthode de l'une quelconque des revendications précédentes, dans laquelle le niveau cellulaire d'ARN messager est déterminé par PCR quantitative, par transfert de Northern ou par analyse par microréseau.

5. La méthode de l'une quelconque des revendications 1 à 3, dans laquelle au moins un polymorphisme d'ACCN1 est détecté au niveau de l'ADN, au niveau de l'ARN ou au niveau de la protéine.

6. La méthode de la revendication 5, dans laquelle au moins un polymorphisme d'ACCN1 est détecté au niveau de l'ADN ou au niveau de l'ARN.

7. La méthode de la revendication 6, dans laquelle le polymorphisme est présent sur la séquence non traduite de l'extrémité 3' de l'ASIC.

8. La méthode de l'une quelconque des revendications 6 et 7, dans laquelle le polymorphisme est un SNP.

9. La méthode de la revendication 8, dans laquelle le SNP est choisi dans le groupe consistant en : SNP rs28936, rs28935, rs28933 et un SNP en position 28364577 bp selon la version de mars 2006 de l'UCSC Genome Browser.

10. La méthode de l'une quelconque des revendications 1 à 3, dans laquelle l'analyse du niveau cellulaire de protéine ACCN1 comprend une technique choisie dans le groupe consistant en techniques immunologiques et spectrométrie de masse.

11. Méthode d'identification d'un composé candidat pour le traitement d'un trouble panique comprenant les étapes consistant à
a. fournir un test de détection d'activité ACCN1,
b. fournir un composé,
c. évaluer l'activité de l'ACCN1 en présence et en absence du composé,
d. vérifier si le composé influe sur l'activité de l'ACCN1 et classer le composé comme composé candidat s'il est capable d'influer sur l'activité de l'ACCN1.
